# EUROPÄISCHE PATENTSCHRIFT

(11) **EP 0 230 628 B1**
(45) Veröffentlichungstag und Bekanntmachung des Hinweises auf die Patenterteilung: **28.02.1990**
(21) Anmeldenummer: 86117719.4
(22) Anmeldetag: 19.12.1986
(51) Int. Cl.: C07D 249/08, A01N 43/653

(54) **Alpha-allylierte Arylalkyltriazole, Verfahren zu ihrer Herstellung und ihre Verwendung als Fungizide**
Alpha-allyl substituted arylalkyltriazoles, process for their preparation and their use as fungicides
Arylalkyltriazoles alpha-allyl-substitués, procédé pour leur préparation et leur utilisation comme fongicides

(30) Priorität: 24.12.1985 DE 3546017
(43) Veröffentlichungstag der Anmeldung: 05.08.1987
(73) Patentinhaber: BASF Aktiengesellschaft, 67063 Ludwigshafen (DE)
(72) Erfinder: Wenderoth, Bernd, Dr., D-6840 Lampertheim (DE); Tuerk, Wolfgang, Dr., D-6700 Ludwigshafen (DE); Rentzea, Costin, Dr., D-6900 Heidelberg (DE); Buschmann, Ernst, Dr., D-6700 Ludwigshaven (DE); Ammermann. Eberhard, Dr., D-6700 Ludwigshaven (DE); Pommer, Ernst-Heinrich, Dr., D-6703 Limburgerhof (DE)

(56) Entgegenhaltungen:
- EP-A- 0 016 323
- EP-A- 0 129 186
- DE-A- 2 638 470
- FR-A- 2 362 133

## Beschreibung

Die vorliegende Erfindung betrifft neue a-allylierte Arylalkyltriazole, Verfahren zu ihrer Herstellung und ihre Verwendung als Fungizide.

Es ist bekannt, daß Triazolderivate wie zum Beispiel das 2,2-Dimethyl-4-(1,2,4-triazol-1-yl)-5-phenyl- pentan-3-on (DE-OS 2 638 470) fungizide Wirksamkeit besitzen. Die Wirkung ist jedoch bei niedrigen Aufwandmengen und Konzentrationen nicht immer ausreichend. Darüber hinaus ist die fungitoxische Wirkung oft mit einer hohen Phytotoxizität verbunden, so daß in den für die Bekämpfung von phytopathogenen Pilzen wie beispielsweise Mehltau, Schorf oder Rostpilzen notwendigen hohen Konzentrationen auch wichtige Kulturpflanzen geschädigt werden.

Es ist ferner bekannt, Triazolalkylderivate, die in Beta-Stellung zum Triazol eine Hydroxygruppe enthalten, oder 1-Allyltriazolderivate als Fungizide zu verwenden (FR-A 2 362 133, EP-A 0 016 323). Durch diese Verbindungen werden die neuen Verbindungen nicht nahegelegt.

Es wurden nun neue Verbindungen gefunden, die neben einer sehr hohen fungitoxischen Wirkung außerdem eine sehr gute Pflanzenvertäglichkeit zeigen.

Gegenstand der Erfindung sind neue Triazolverbindungen der Formel 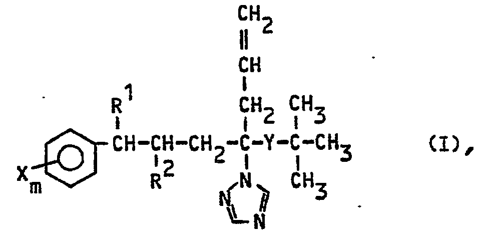 in aer
R¹ und R² verschieden oder gleich sind und Wasserstoff oder Alkyl mit 1-3 C-Atomen bedeuten,
X Wasserstoff-, Halogen-, Cyano-, Nitro-, C₁-C₄-Alkyl, C₁-C₄-Alkoxy und
m eine ganze Zahl von 1-5 bedeutet, wobei X gleich oder verschieden sein kann, wenn m größer als 1 ist und
y eine -CO- oder -CH(OH)-Gruppe bedeutet.

Bevorzugt werden Triazolverbindungen der Formel I, worin R1 und R² Wasserstoff oder Methyl, X Wasserstoff, Halogen, Methyl oder Methoxy, m 1 oder 2 und Y CHOH bedeuten.

Die neuen Verbindungen der Formel 1 fallen bei ihrer Herstellung als Enantiomeren- bzw. Diastereomerengemische an, die in der üblichen Weise, z.B. durch Kristallisation oder Chromatographie, in die einzelnen Komponenten getrennt werden können. Die einzelnen isomeren Verbindungen und ihre Gemische werden von der Erfindung umfaßt. Für die Anwendung der neuen Verbindungen als Fungizide ist jedoch eine Trennung der Enantiomeren oder Diastereomeren normalerweise nicht erforderlich.

Die neuen Verbindungen der Formel I, in der Y den Rest -CO- bedeutet, lassen sich herstellen, indem man Ketone der Formel II 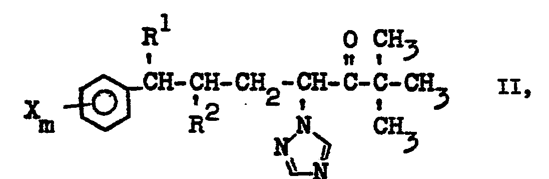 wobei Xₘ, R1 und R2 die in Anspruch 1 genannte Bedeutung haben oder deren Alkalienolate mit einem Allylhalogenid der Formel III 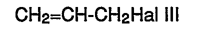 in der Hal für Chlor oder Brom steht, gegebenenfalls in Gegenwart eines Lösungs- oder Verdünnungsmittels und/oder anorganischer Basen bei Temperaturen zwischen 0 und 200_{°}C umsetzt. Die Ketone der Formel II sind bekannte Verbindungen (DE-OS 33 21 422), die nach dem dort beschriebenen Verfahren hergestellt werden können.

Die so erhaltenen Ketone der Formel I, in der Y für eine CO-Gruppe steht, lassen sich gegebenenfalls reduzieren, z. B. durch Umsetzung mit einer geeigneten Grignard-Verbindung, wie n-Propylmagnesiumbromid in Gegenwart eines geeigneten aprotischen Lösungsmittels, wie Ether oder Tetrahydrofuran, bei Temperaturen von O_{°}C bis zum Siedepunkt des Lösungsmittels und anschließende Hydrolyse des sekundären Magnesiumalkoholats in üblicher Weise oder z. B. durch Einwirkung von komplexen Hydriden, bevorzugt Natriumborhydrid in Gegenwart eines polaren Lösungsmittels, z. B. eines Alkohols, bevorzugt Methanol oder Ethanol bei Temperaturen zwischen 0 und 100_{°}C und anschließender Hydrolyse mit wäßrigen Basen oder Säuren oder durch Einwirkung von Wasserstoff in Gegenwart eines Hydrierungskatalysators wie Platin oder Raney-Nickel und in Gegenwart eines polaren Lösungsmittels wie Methanol, Ethanol oder Ethylacetat bei Temperaturen zwischen 20 und 100_{°}C und Drücken von 1 bis 100 bar.

Die Herstellung der neuen Verbindungen der Formel 1 wird durch folgende Beispiele erläutert:

### Beispiel 1 (Verb.-Nr. 22 der Tabelle)

Zu einer unter Stickstoff gerührten Suspension von 6,8 g (227 mmol) Natriumhydrid in 150 ml absolutem Dimethyiformamid werden 63 g (118 mmol) 2,2,6-Trimethyl-4-(1,2,4-triazol-1-yl)-7-(4-chlorphenyl)-heptan-3-on in 150 ml absolutem Dimethylformamid bei Raumtemperatur langsam zugetropft. Nach einstündigem Nachrühren bei 50_{°}C werden anschließend bei Raumtemperatur 17,9 ml (207 mmol) Allylbromid zugetropft. Danach wird 2 Tage bei Raumtemperatur gerührt. Anschließend wird durch vorsichtige H₂0-Zugabe überschüssiges Natriumhydrid zerstört und mit CH₂CI₂ mehrmals extrahiert. Die organische Phase wird nun mehrmals mit Wasser gewaschen, über NazS04 getrocknet und eingeengt. Nach der Destillation im Ölpumpenvakuum (Kpo,oi= 200_{°}C) werden 53 g (75 %) 2,2,6-Trimethyl-4-(2-propen-1-yl)-4-(1,2,4-triazol-1-yl)-7-(4-chlorphenyl)-heptan-3-on (Verbindung Nr. 22) erhalten.

### Beispiel 2. (Verb.-Nr. 23 der Tabelle)

Zu einer Lösung von 21 g (56 mmol) 2,2,6-Trimethyl-4-(2-propen-1-yl)-4-(2-propen-1-yl)-4(1,2,4-triazol-1-yl)-7(4-chlorphenyl)-heptan-3-on in 300 ml absolutem Methanol werden zwischen 0 und +5_{°}C 2,6 g (70 mmol) Natriumborhydrid portionsweise zugegeben. Nach fünfstündigem Rühren unter Rückfluß wird eingeengt und der Rückstand zwischen 400 ml Ether und 200 ml Wasser verteilt, die etherische Phase dreimal mit 200 ml Wasser gewaschen, über Natriumsulfat getrocknet und eingeengt. Das anfallende Öl wird mit n-Pentan zur Kristallisation gebracht; die Kristalle werden abgesaugt und getrocknet.Man erhält 11 g (52 %) 2,2,6-Trimethyl-4-(2-propen-1-yl)-4-(1,2,4-triazol-1-yl)-7-(4-chlorphenyl)-heptan-3-ol (Verbindung Nr. 23) als weiße Kristalle (Fp = 98 - 100°C).

In entsprechender Weise werden die in der folgenden Tabelle aufgeführten Verbindungen erhalten.

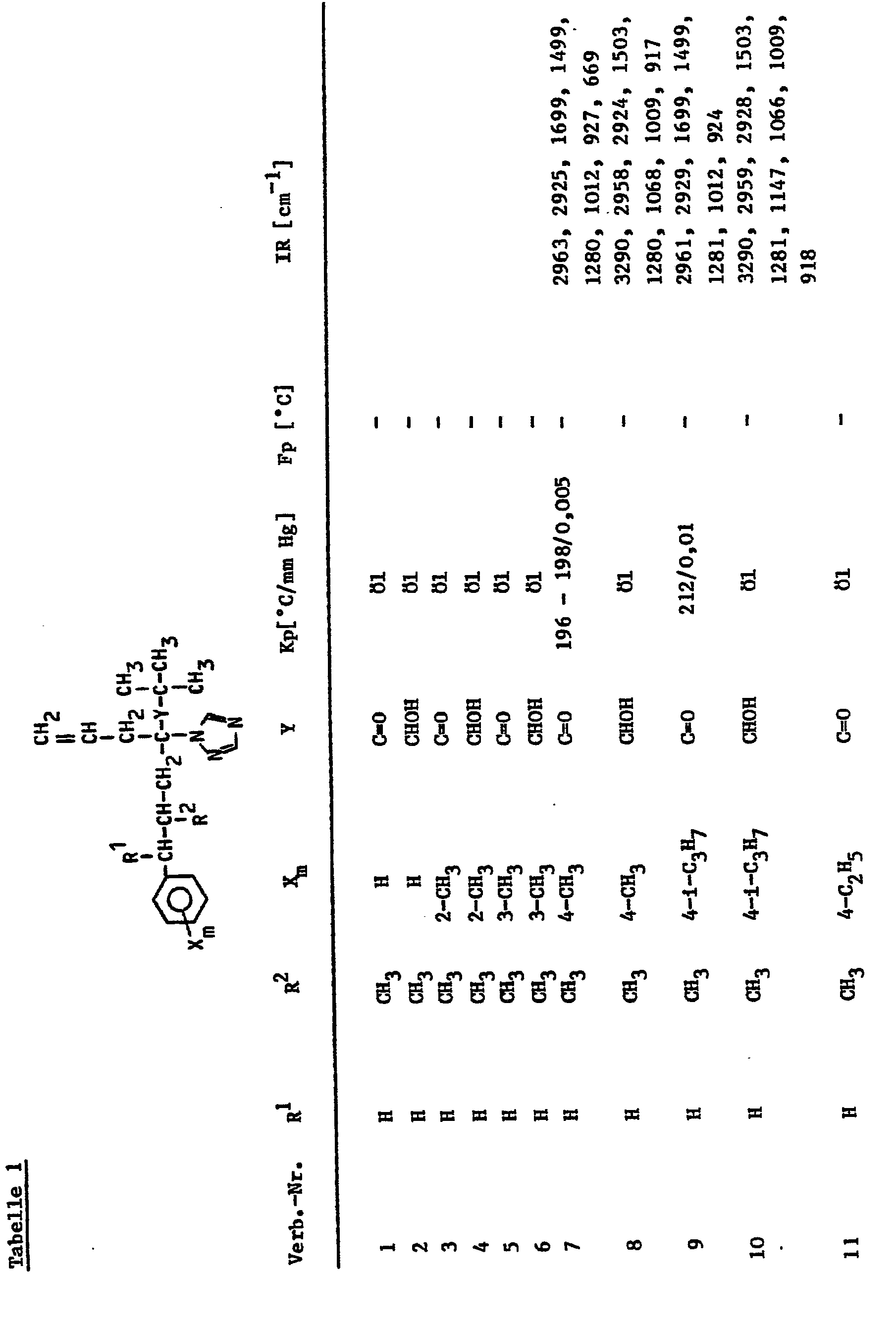

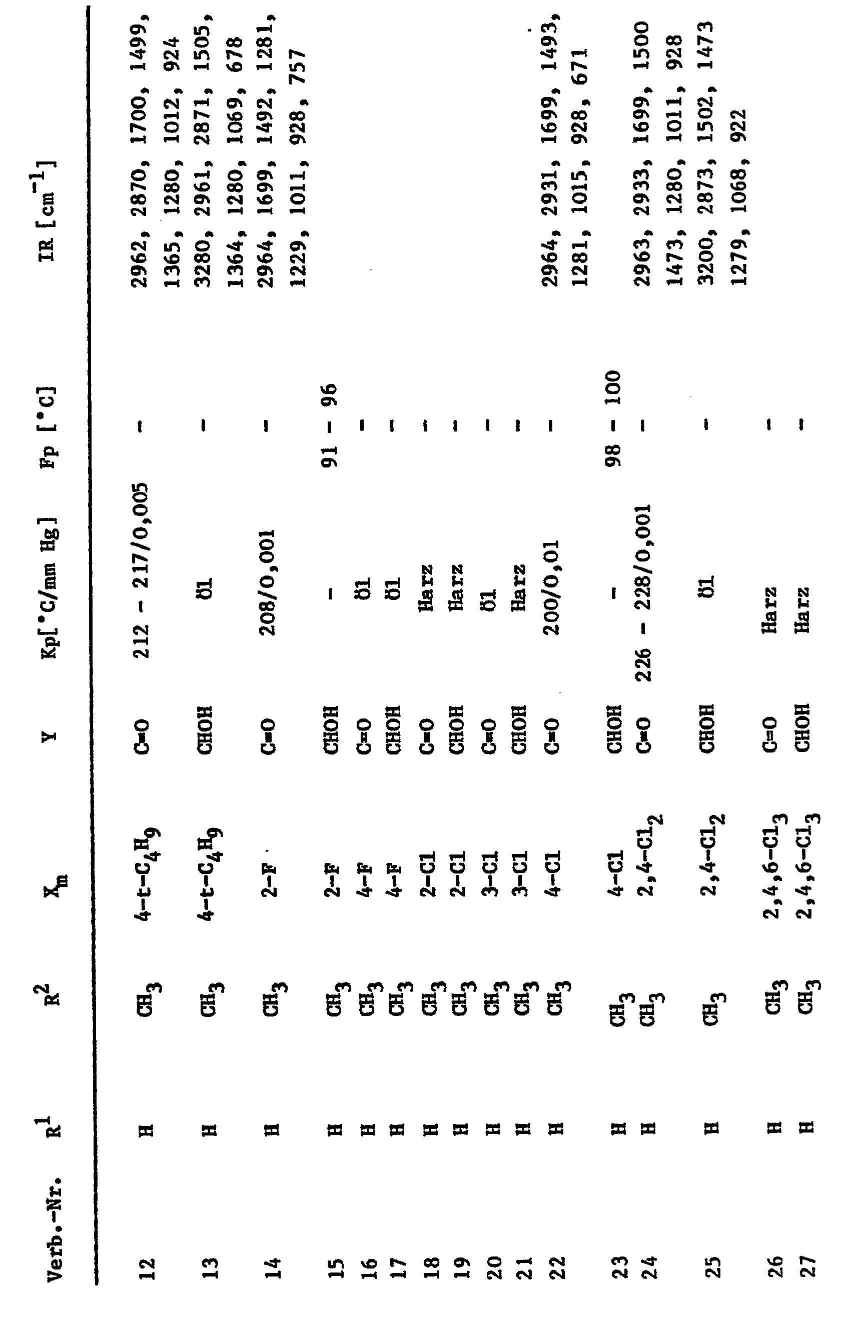

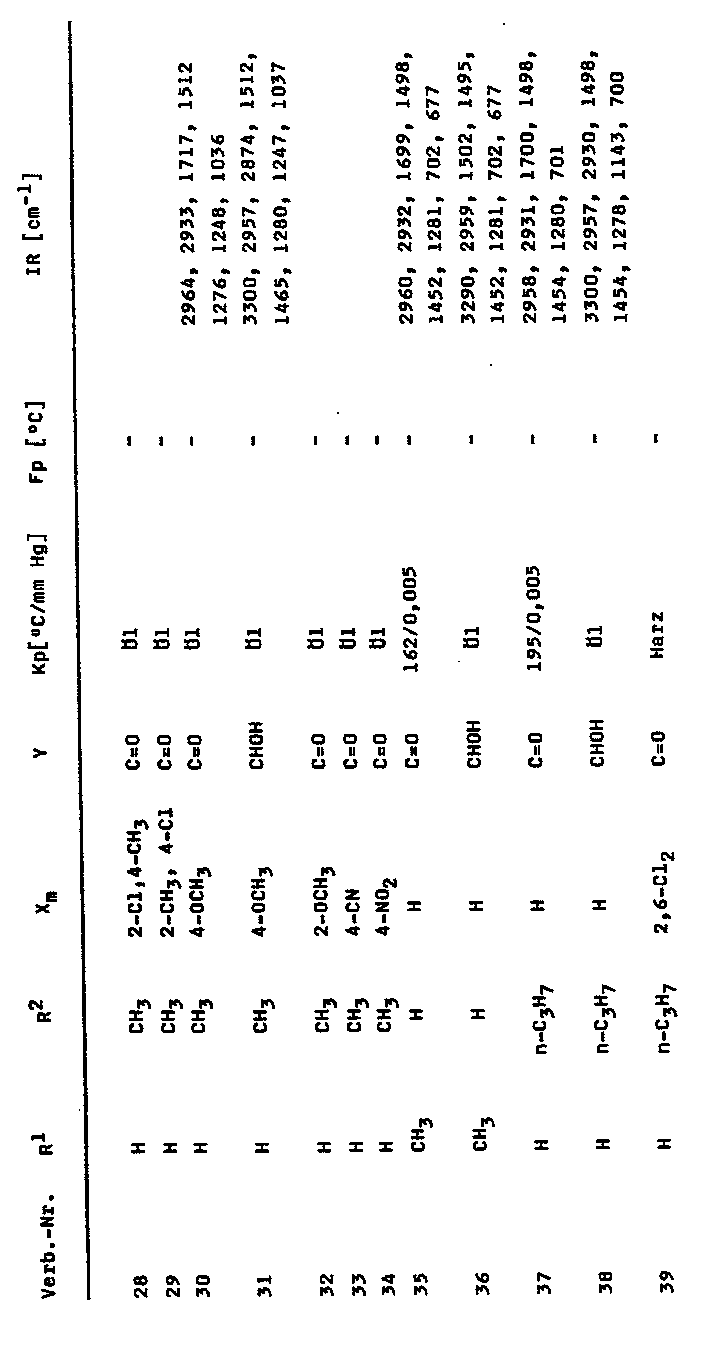

Die neuen Verbindungen zeichnen sich, allgemein ausgedrückt, durch eine hervorragende Wirksamkeit gegen ein breites Spektrum von pflanzenpathogegen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja,-Kaffee, Zuckerrohr, Obst und Zierpflanzen im Gartenbau, Weinbau sowie Gemüse - wie Gurken, Bohnen und Kürbisgewächse - .

Die neuen Verbindungen sind insbesondere geeignet zur Bekämpfung folgender Pflanzenkrankheiten:
Erysiphe graminis (echter Mehltau) in Getreide,
Erysiphe cichoracearum an Kürbisgewächsen,
Podosphaera leucotricha an Äpfeln,
Uncinula necator an Reben,Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Einige der Verbindungen eignen sich auch zur Bekämpfung hautpathogener Pilze, z.B. Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:
I. Man vermischt 90 Gewichtsteile der Verbindung Nr. 8 mit 10 Gewichtsteilen N-Methyl-alpha-pyrrolidon und erhält eine Lösung, die zur Anwendung in Form kleinster Tropfen geeignet ist.
11. 10 Gewichtsteile der Verbindung Nr. 10 werden in einer Mischung gelöst, die aus 80 Gewichtsteilen Xylol, 10 Gewichtsteilen des Anlagerungsproduktes von 8 bis 10 Mol Ethylenoxid an 1 Mol Olsäure-N-fonsäure und 5 Gewichtsteilen des Anlagerungsproduktes von 40 Mol -monoethanolamid, 5 Gewichtsteilen Calciumsalz der Dodecylbenzolsul-Ethylenoxid an 1 Mol Ricinusöl besteht. Durch Ausgießen und feines Verteilen der Lösung in Wasser erhält man eine wäßrige Dispersion.
111. 20 Gewichtsteile der Verbindung Nr. 15 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gewichtsteile der Verbindung Nr. 12 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilender Lösung in Wasser erhält man eine wäßrige Dispersion.
V. 80 Gewichtsteile der Verbindung Nr. 13 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.
Vf. 3 Gewichtsteile der Verbindung Nr. 23 werden mit 97 Gewichtsteilen feinteiligem Kaolin innig vermischt. Man erhält auf diese Weise ein Stäubemittel, das 3 Gew.% des Wirkstoffs enthält.
VII. 30 Gewichtsteile der Verbindung Nr. 24 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gewichtsteile der Verbindung Nr. 25 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion. Durch Verdünnen mit Wasser erhält man eine wäßrige Dispersion.
IX. 20 Teile der Verbindung Nr. 31 werden mit 2 Teilen Calciumsalz der Dodecylbenzolsulfonsäure, 8 Teilen Fettalkoholpolyglykolether, 2 Teilen Natriumsalz eines Phenoisulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat
Tetramethylthiuramdisulfide,
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol
2-(Furyl)-(2)-benzimidazol
2-(Thiazotyi-(4))-benzimidazo!,
N-(1,1,2,2-Tetrachlorethylthio)-tetrahydrophthalimid N-Trichlormethylthio-tetrahydrophthalimid
N-Trichlormethylthio-phthalimid
N-Dichlortluormethylthio-N',N'-dimethyl-N-phenyl-schwefelsäurediamid
5-Ethoxy-3-trichlormethyl-1,2,3-thiadiazol
2-Rhodamethylthiobenzthiazol
1,4-Dichlor-2,5-dimethoxybenzol
4-(2-Chlorphenylhydrazono)-3-methyl-5-isoxazolon
Pyridin-2-thio-l-oxid
8-Hydroxychinolin bzw. dessen Kupfersalz
2,3-Dihydro-5-carboxanilido-6-methyl-1,4-oxathiir
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-lod-benzoesäure-anilid
N-Formyl-N-morpholin-2,2,2-trichlorethylacetal
Piperazin-1,4-diylbis-(1 -(2,2,2-trichlor-ethyl)-formamid
1-(3,4-Dichloranilino)-1 -formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2,-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat,
3-(3-(3,5-Dimethyl-2-oxycyclohexyl)-2-hydroxyethyl)-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino)-acetamid
1-(2-(2,4-Dichlorphenyl)-pentyl)-1 H-1,2,4-triazol
2,4-Difluor-alpha-(1 H-1,2,4-triazolyl-1-methyl)-benzhydrylalkohol

Die folgenden Versuche wurden als Vergleich der bekannte Wirkstoff 2,2-Dimethyl-4-(1,2,4-triazol-1-yl)-5-phenyl-pentan-3-on (A) verwendet.

### Anwendungsbeispiel 1

### Wirksamkeit gegen Gurkenmehltau

Blätter von in Töpfen gewachsenen Gurkenkeimlingen der Sorte "Chinesische Schlange" werden im Zweiblattstadium mit einr Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22_{°}C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß bei der Anwendung als 0,025%ige Spritzbrühe beispielsweise die Verbindungen 8, 10, 12, 13, 15, 23, 24, 25, 31 und 36 eine bessere fungizide Wirkung haben (97%) als der bekannte Wirkstoff A (60%).

### AnwendunasbeisDiel 2

### Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22_{°}C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22_{°}C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß bei der Anwendung als 0,025%ige Spritzbrühe beispielsweise die Verbindungen 8, 22, 31 und 36 eine bessere fungizide Wirkung haben (97%) als der bekannte Wirkstoff A (90 %).

### Anwendungsbeispiel 3

### Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24_{°}C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß bei der Anwendung als 0,05%ige Spritzbrühe beispielsweise die Verbindungen 15, 25 und 36 eine bessere fungizide Wirkung haben (90 %) als der bekannte Wirkstoff A (60 %).

## Patentansprüche

1. Verbindungen der Formel 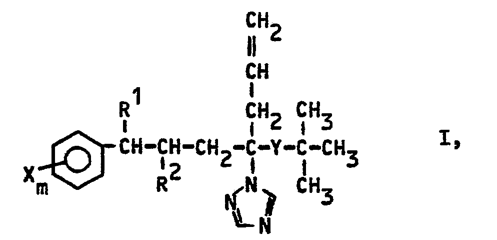 in der
R¹ und R² verschieden oder gleich sind und Wasserstoff oder Alkyl mit 1-3 C-Atomen bedeuten,
X Wasserstoff, Halogen, Cyano, Nitro, C₁-C₄-Alkyl, C₁-C₄-Alkoxy und
m eine ganze Zahl von 1 bis 5 bedeutet, wobei X gleich oder verschieden sein kann, wenn m größer als 1 ist und
Y eine -CO- oder -CH(OH)-Gruppe bedeutet.

2. Verbindungen der Formel gemäß Anspruch 1, in der
Xₘ Wasserstoff, 2-Fluor-, 4-Fluor-, 2-Chlor-, 3-Chlor-, 4-Chlor-, 2,4-Dichior-, 2,6-Dichlor-, 2,4,6-Trichlor-, 2-Chlor-4-methyl-, 2-Methyl-4-chlor-, 2-Methyl-, 3-Methyl-, 4-Methyl-, 4-Ethyl-, 4-lsopropyl-, 4-tert.-Butyl-, 2-Methox^{y-}, 4-Methoxy-, 4-Cyano-, 4-Nitro-,
R¹ Wasserstoff oder Methyl,
R² Wasserstoff, Methyl oder Propyl,
Y eine -CO- oder-CH(OH)-Gruppe bedeutet.

3. Mittel zur Bekämpfung von Pilzen, enthalten eine oder mehrere Verbindungen gemäß Anspruch 1.

4. Mittel zur Bekämpfung von Pilzen, enthalten eine oder mehrere Verbindungen der Formel 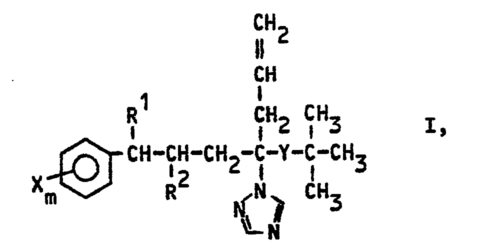 in der
R¹ und R² verschieden oder gleich sind und Wasserstoff oder Alkyl mit 1-3 C-Atomen bedeuten,
X Wasserstoff, Halogen, Cyano, Nitro, C₁-C₄-Alkyl, C₁-C₄-Alkoxy und
m eine ganze Zahl von 1 bis 5 bedeutet, wobei X gleich oder verschieden sein kann, wenn m größer als 1 ist und
Y eine -CO- oder -CH(OH)-Gruppe bedeutet,
und eine flüssigen oder festen Trägerstoff.

5. Verwendung von Verbindungen gemäß Anspruch 1 zur Bekämpfung von Pilzen.

6. Verfahren zur Bekämpfung von Pilzen, dadurch aekennzeichnet, daß man eine Verbindung der Formel 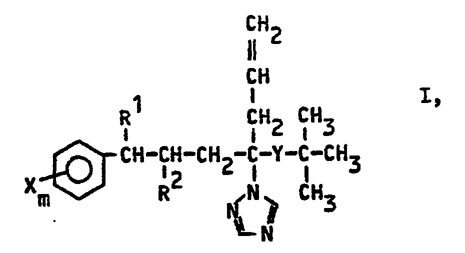 in der
R¹ und R² verschieden oder gleich sind und Wasserstoff oder Alkyl mit 1-3 C-Atomen bedeuten,
X Wasserstoff, Halogen, Cyano, Nitro, C₁-C₄-Alkyl, C₁-C₄-Alkoxy und
m eine ganze Zahl von 1 bis 5 bedeutet, wobei X gleich oder verschieden sein kann, wenn m größer als 1 ist und
Y eine -CO- oder-CH(OH)-Gruppe bedeutet, auf diese oder auf durch Pilzbefall bedrohte Flächen, Pflanzen oder Saatgüter einwirken läßt.

7. Verfahren zur Herstellung eines fungiziden Mittels, dadurch aekennzeichnet, daß man ein Triazolylderivat der Formel I gemäß Anspruch 1 mit festen oder flüssigen Trägerstoffen sowie gegebenenfalls mit einem oder mehreren oberflächenaktiven Mitteln mischt.

8. Verbindung gemäß Anspruch 1, worin R¹ und R² Wasserstoff oder Methyl, X Wasserstoff, Halogen, Methyl oder Methoxy, m 1 oder 2 und Y CHOH bedeuten.

## Claims

1. A compound of the formula I 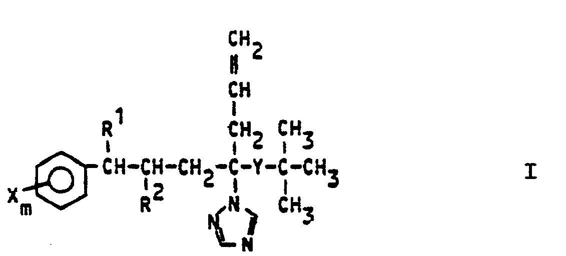 where R¹ and R² are identical or different and are each hydrogen or alkyl of 1 to 3 carbon atoms, X is hydrogen, halogen, cyano, nitro, C₁-C₄-alkyl or C₁-C₄-alkoxy and m is an integer from 1 to 5 and X may be identical or different when m is greater than 1, and Y is -CO- or -CH-(OH)-.

2. A compound of the formula I as claimed in claim 1, wherein Xm is hydrogen, 2-fluoro-, 4-fluoro-, 2-chloro-3-chloro-, 4-chloro-, 2,4-dichloro-, 2,6-dichloro-, 2,4,6-trichloro-, 2-chloro-4-methyl-, 2-methyl-4-chloro-2-methyl-, 3-methyl-, 4-methyl-, 4-ethyl-, 4-isopropyl-4-tert-butyl-, 2-methoxy-, 4-methoxy-, 4-cyano- or 4-nitro-, R¹ is hydrogen or methyl, R² is hydrogen, methyl or propyl and Y is -CO- or -CH-(OH)-.

3. A fungicide containing one or more compounds as claimed in claim 1.

4. A fungicide containing one or more compounds of the formula I 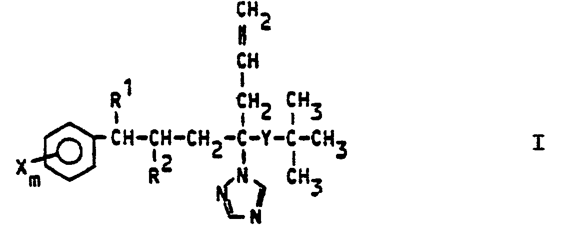 where R¹ and R² are identical or different and are each hydrogen or alkyl of 1 to 3 carbon atoms, X is hydrogen, halogen, cyano, nitro, C₁-C₄-alkyl or C₁-C₄-alkoxy and m is an integer from 1 to 5 and X may be identical or different when m is greater than 1, and Y is -CO- or -CH-(OH)-, and a liquid or solid carrier.

5. Use of a compound as claimed in claim 1 for controlling fungi.

6. A method for controlling fungi, wherein a compound of the formula I 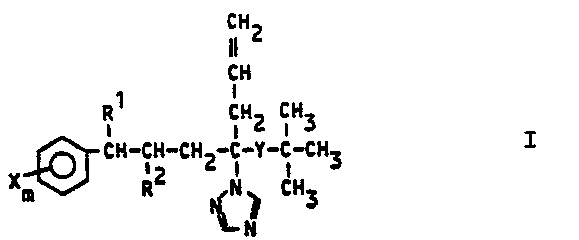 where R¹ and R² are identical or different and are each hydrogen or alkyl of 1 to 3 carbon atoms, X is hydrogen, halogen, cyano, nitro, C₁-C₄-alkyl or C₁-C₄-alkoxy and m is an integer from 1 to 5 and X may be identical or different when m is greater than 1, and Y is -CO- or -CH-(CH)-, is allowed to act on the fungi or on areas, plants or seed threatened by fungal attack.

7. A process for the preparation of a fungicide, wherein a triazolyl derivative of the formula I as claimed in claim 1 is mixed with solid or liquid carriers and, if required, with one or more surfactants.

8. A compound as claimed in claim 1, wherein R¹ and R² are each hydrogen or methyl, X is hydrogen, halogen, methyl or methoxy, m is 1 or 2 and Y is CHOH.

## Revendications

1. Composés de formule 1 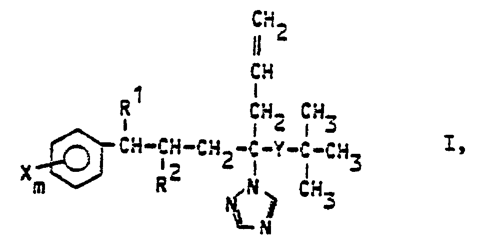 dans laquelle
R¹ et R² sont identiques ou différents et représentent hydrogène, halogène, cyano, nitro, alkyle en C₁₋₃,
X, hydrogène, halogène, cyano, nitro, alkyle en C₁₋₄ alcoxy en C₁₋₄ et
m, un nombre entier de 1 à 5, les X pouvant être identiques ou différents, si m est supérieur à 1, et
Y représente un groupe CO ou -CH(OH).

2. Composés de formule 1 selon la revendication 1, dans laquelle
Xm représente hydrogène 2-fluoro-, 4-fluoro-, 2-chloro-, 3-chloro-, 4-chloro-, 2,4-dichloro-, 2,6-dichloro-, 2,4, 6-trichloro-, 2-chloro-4-méthyl-, 2-méthyl-4-chloro-, 2-méthyl-, 3-méthyl-, 4-méthyl-, 4-ethyl-, 4-isopropyl-, 4-tert.-butyl-, 2-methoxy-, 4-methoxy-, 4-cyano-, 4-nitro-,
R¹ représente hydrogène ou méthyle,
R2 représente hydrogène, méthyle ou propyle,
Y un groupe -CO ou -CH(OH).

3. Moyen de lutte contre les champignons contenant un ou plusieurs composés selon la revendication 1.

4. Moyen de lutte contre les champignons contenant un ou plusieurs composés de formule 1 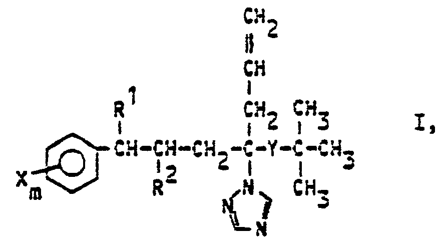 dans laquelle
R¹ et R² sont identiques ou différents et représentent hydrogène, halogène, cyano, nitro, alkyle en Cₗ-3,
X, hydrogène, halogène, cyano, nitro, alkyle en C₁₋₄ alcoxy en C₁₋₄ et
m, un nombre entier de 1 à 5, les X pouvant être identiques ou différents, si m est supérieur à 1, et
Y représente un groupe CO ou -CH(OH).
et un support liquide ou solide.

5. Utilisation de composés selon la revendication 1 pour lutter contre les champignons.

6. Procédé de lutte contre les champignons, caractérisé par le fait que l'on fait agir sur ceux-ci, ou sur les surfaces, plantes ou semences menacées par les champignons, un composé de formule 1 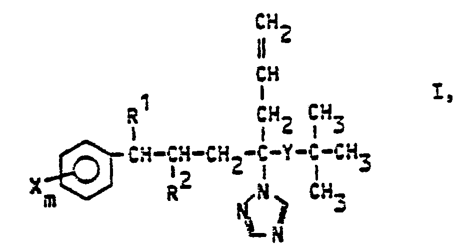 dans laquelle
R¹ et R² sont identiques ou différents et représentent hydrogène, halogène, cyano, nitro, alkyle en C₁₋₃,
X, hydrogène, halogène, cyano, nitro, alkyle en C₁₋₄ alcoxy en C₁₋₄ et
m, un nombre entier de 1 à 5, les X pouvant être identiques ou différents, si m est supérieur à 1, et
Y représente un groupe CO ou -CH(OH).

7. Procédé de préparation d'un agent fongicide caractérisé par le fait que l'on mélange un dérivé tria- zolyle de formule I selon la revendication 1 avec des substances supports solides ou liquides, ainsi qu'avec éventuellement un ou plusieurs agents tensio-actifs.

8. Composé selon la revendication 1, dans lequel R¹ et R² représentent hydrogène ou méthyle, X, hydrogène, halogène, méthyle ou méthoxy, m, 1 ou 2 et Y, CHOH.
